Europäisches Patentamt

European Patent Office

Office européen des brevets

(11) Publication number: **0 295 619**
**A2**

(12) **EUROPEAN PATENT APPLICATION**

(21) Application number: 88109435.3

(22) Date of filing: 14.06.88

(51) Int. Cl.⁴: **A61K 31/557**

The title of the invention has been amended (Guidelines for Examination in the EPO, A-III, 7.3).

(30) Priority: 16.06.87 CS 4415/87

(43) Date of publication of application:
**21.12.88 Bulletin 88/51**

(84) Designated Contracting States:
**BE CH DE FR GB IT LI LU NL SE**

(71) Applicant: **SPOFA spojené podniky pro zdravotnickou vyrobu**
**No 11a Husinecká**
**Praha 3(CS)**

(72) Inventor: **Kral, Josef, Dipl.Ing.**
**Chotoun 55**
**Pohori(CS)**
Inventor: **Sevcik, Bohumil Prof.MVDr. DrSc.**
**Chotoun 79**
**Pohori(CS)**
Inventor: **Borovicka, Antonin, Dipl.Ing.**
**Hlavni 279**
**Davle(CS)**
Inventor: **Bilek, Petr, Dipl.Ing.**
**Kyjevska 14**
**Praha 6(CS)**
Inventor: **Lamprechtova, Svetlana, Dipl.Ing.**
**U druzstva REPO 9**
**Praha 4(CS)**

(74) Representative: **Patentanwälte Beetz sen. - Beetz jun. Timpe - Siegfried - Schmitt-Fumian- Mayr**
**Steinsdorfstrasse 10**
**D-8000 München 22(DE)**

(54) **Cloprostenol for the control of ovarian functions in farm animals.**

(57) The invention relates to a method and a preparation for varian functions in farm animals on the basis of an optically active isomer of cloprostenol in which at least 99 % of the prostanoic constituent consist of D-cloprostenol. Doses of only 40 to 150 μg cloprostenol are sufficient, corresponding to only 10 to 30 % of the usual dose of cloprostenol racemate. Administration is effected by intramuscular injection. The organism of the animal is not burdened by a foreign substance, and residues in milk of the first milking after administration decrease rapidly below a detectable limit.

## Method and preparation for the control of ovarian functions in farm animals

The present invention relates to a method and a pharmaceutical composition for the control of ovarian functions in farm animals by means of a highly active substance of prostanoid F group.

The control of ovarian functions in farm animals has become a common biotechnical practice in recent years. A principle of the invention consists in limiting the functional activity of the corpus luteum (CL), a periodic gland on the ovary. If a convenient prostanoid is given to a cycling cow (heifer), the sexual cycle shortens enabling thus its oestrous phase to be planned for the convenient term established beforehand. A luteolytic prostanoid administered to a sow on the last days of gestation induces the onset of farrowing. Analogous substances are used for recovery of the pathological condition of ovaries with persisting CL.

At present prostaglandin preparations are used for treatment for all the above mentioned indications. In most of the cases, cloprostenol, a synthetic analogue of the natural prostaglandin $F_2$ alpha, is their active constituent. Doses of 500μg cloprostenol for cows and 175 μg for sows are used to achieve the desired effects.

Cloprostenol is a racemic mixture of optically active isomers, D-cloprostenol (dextrorotatory) and L-cloprostenol (leavorotatory), at a ratio of approximately 1:1. Tests of the individual biological activity of the pure separated isomers revealed quite unexpectedly that merely D-cloprostenol is the carrier of luteolytic activity. L-cloprostenol is either CL indifferent, or stimulates the CL function under particular, not quite clear conditions. Hence, its activity may be antagonistic to the effect of D-cloprostenol. Accordingly, simultaneous administration of these two functional antagonists is inconvenient. D-cloprostenol administered individually induces luteolysis in cows at a dose of 125 μg. Under in-process conditions identical results were obtained after administration of 500 μg cloprostenol racemate and 150 μg D-cloprostenol.

It is the object of the present invention to provide a method and a pharmaceutical composition for the control of ovarian functions in farm animals on the basis of cloprostenol, which has a more specific and higher activity.

The above object is achieved according to the claims.

The method according to the invention consists in administering a prostanoic active ingredient on the basis of D-cloprostenol, preferably having a content of at least 99 % of D-cloprostenol.

The pharmaceutical composition according to this invention on the basis of cloprostenol are characterized in that the prostanoic constituent essentially consists of D-cloprostenol and preferably ≧ 99% of the prostanoic constituent consist of D-cloprostenol.

Cloprostenol is rapidly degraded and metabolized in the organism, however, despite of that, residues of about 0,1 ng/ml are detected in the milk of the first milking after administration of a dose of 500 μg. If the dependence between the dose and the residual level is linear, no residue will be detectable more than 8 h after administration of the preparation according to this invention.

The disadvantages of previous methods of controlling the ovarian functions in farm animals, particularly by means of racemic cloprostenol mixture, have been overcome by the preparation according to this invention wherein at least 99 % of its prostanoic constituent accounts for D-cloprostenol. The administration to farm animals is conducted by intramuscular injection of 40 to 150 μg D-cloprostenol per female animal.

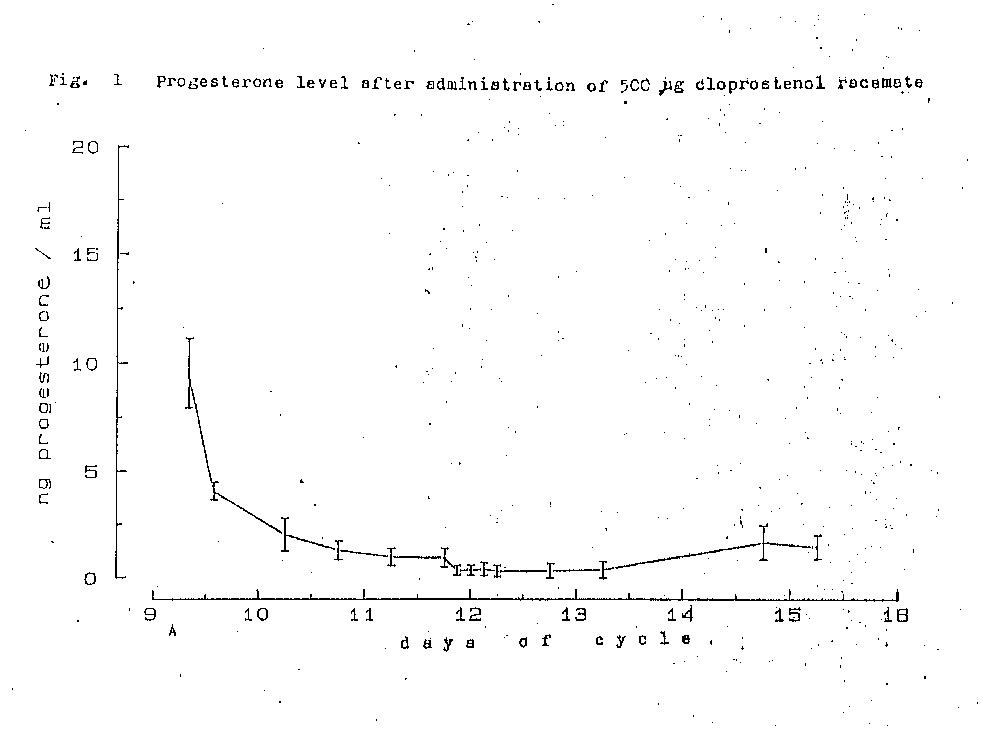

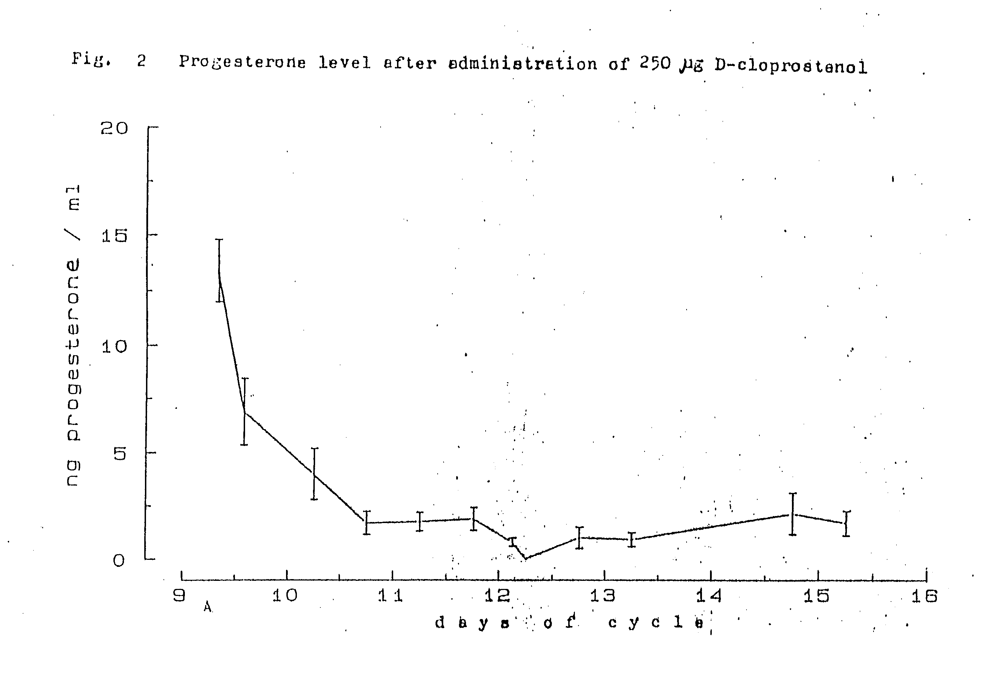

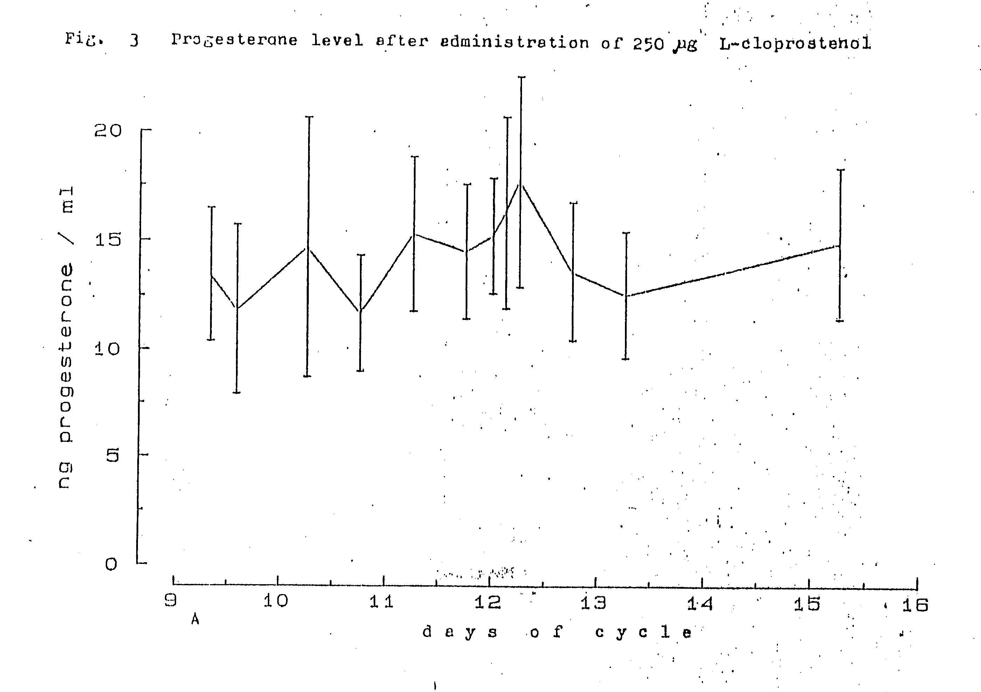

The activity of the preparation according to the invention is documented by the enclosed Figures 1 to 4. Fig. 1 is a graph illustrating the mean decrease of blood plasma progesterone concentration in heifers following intramuscular administration of 500 μg cloprostenol racemate. The characteristic of the curve indicates that shortly after administration the progesterone level decreases below 1 ng/ml, i.e. the value necessary for the onset of the oestrous phase of the cycle. Fig. 2 is the same kind of graph as Fig. 1, showing progesterone concentration values after administration of 150 μg of D-cloprostenol; the decrease characteristic is analogous to that after administration of the racemate (Fig. 1). On the other hand, Fig. 3 shows the influence of L-cloprostenol on the blood plasma level. As is evident from Fig. 3, no decrease in progesterone level occurs after administration of 250 μg L-cloprostenol. These findings confirm that the D-isomer of cloprostenol is the only carrier of luteolytic activity, and, accordingly, can be used independently.

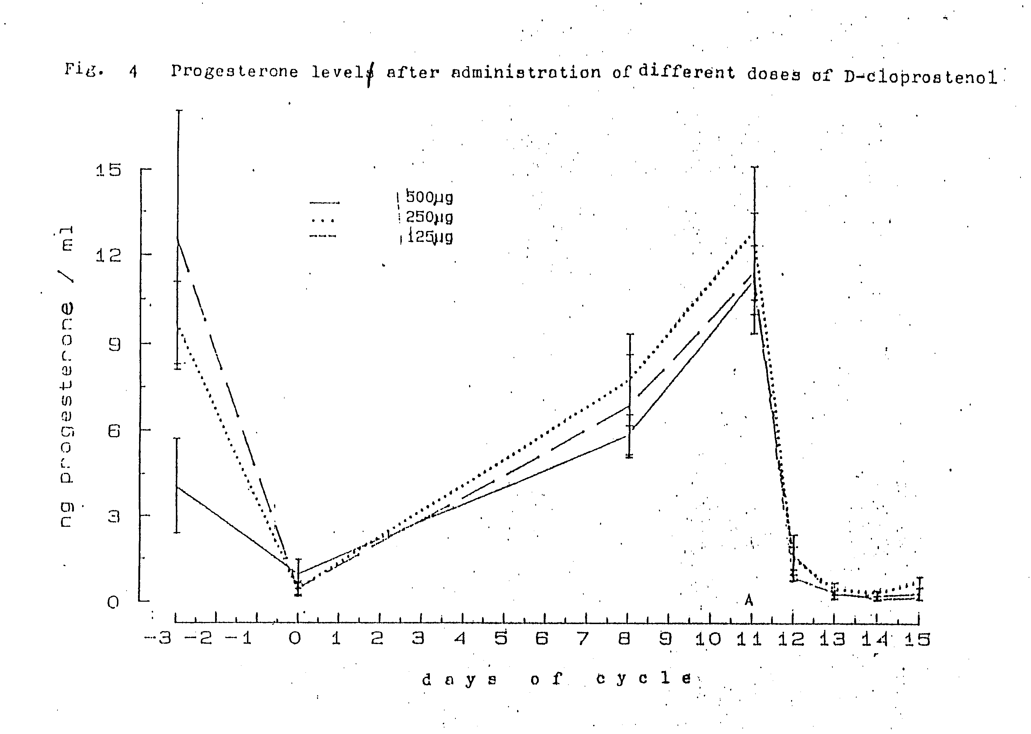

Fig. 4 represents the effects of different intramusculary doses of D-cloprostenol on the function of the corpus luteum in cows of 600 to 650 kg. It may be seen that progesterone secretion ceased also after a dose of 125 μg.

Test results obtained with the composition of the invention are summarized in Tables 1 to 3. Table 1 shows identical biological activities of 500 μg cloprostenol racemate and 150 μg D-cloprostenol used for the control of the sexual cycle in cows. A number of repeated administrations did not influence the activity of the substance which depends on the phase of the sexual cycle at the time of administration. The onset of heat and conception rate were decisive factors.

Table 1

| Induction of heat in cows by cloprostenol racemate and D-cloprostenol | | | | | |
|---|---|---|---|---|---|
| | Number of cows | Number of doses | | Conception after 1st insemination | |
| | | 1 | 2 | ks | % |
| Cloprostenol racemate | 76 | 52 | 24 | 38 | 50 |
| D-cloprostenol | 69 | 51 | 18 | 36 | 52,17 |

Table 2 summarizes results of inducing farrowings in sows. A dose of 45 $\mu$g D-cloprostenol induced the commence of farrowing at a time comparable with a higher dose or with 175 $\mu$g cloprostenol racemate.

Table 2

| Induction of farrowings in sows by cloprostenol racemate and D-cloprostenol | | | | |
|---|---|---|---|---|
| | number of sows | **Parturition** | | live-born piglets (%) |
| | | Onset after administr. (h) | Duration (h) | |
| Cloprostenol racemate 175 $\mu$g | 96 | 20,61 | 3,53 | 87,2 |
| D-cloprostenol 75 $\mu$g | 106 | 21,31 | 3,79 | 88,5 |
| D-cloprostenol 45 $\mu$g | 46 | 22,39 | 4,23 | 87,2 |

Table 3 represents results obtained after administration of cloprostenol to cows that failed to be mated within 65 days after calving. After repeated observations, the cows were assigned to a group with persisting corpus luteum and to a group cycling without any external manifestations of heat.

In both groups approximately a half of the animals were given cloprostenol racemate and a half D-cloprostenol. The results obtained were equal, thus demonstrating that 150 $\mu$g D-cloprostenol might induce full heat in some cases of non-physiological conditions of sexual organs.

Table 3

| Induction of physiological heat in cows | | | | | | | |
|---|---|---|---|---|---|---|---|
| Result | Substance | number of treated animals | inseminated | | conception within 60 days | | mean days till conception |
| | | | heats | % | heats | % | |
| Anoestrus with persisting CL | cloprostenol racemate | 14 | 14 | 100 | 10 | 71,4 | 17,7 |
| | D-cloprostenol | 13 | 13 | 100 | 10 | 76,9 | 18,7 |
| Anoestrus without pathological finding | cloprostenol racemate | 23 | 19 | 82,6 | 12 | 52,2 | 23,5 |
| | D-cloprostenol | 25 | 20 | 80,0 | 15 | 60 | 21,1 |

## Claims

1. A method for controlling the ovarian functions in farm animals by administering an active constituent on the basis of cloprostenol, **characterized** in that D-cloprostenol is used.

2. The method according to claim 1, characterized in that a prostanoic active ingredient having a content of at least 99 % of D-cloprostenol is used.

3. The method according to claim 1 or 2, characterized in that D-cloprostenol is administered in single doses of 40 to 150 μg.

4. The method according to one of claims 1 to 3, characterised in that D-cloprostenol is administered by intramuscular injection.

5. A pharmaceutical composition for the control of ovarian functions in farm animals on the basis of cloprostenol, characterized by D-cloprostenol as prostanoic constituent.

6. The pharmaceutical composition according to claim 5, characterized in that the prostanoic constituent has a content of at least 99 % of D-cloprostenol.

7. The pharmaceutical composition according to claim 5 or 6, characterized in that it is formulated in the galenic form of dosage units comprising 40 to 150 μg of D-cloprostenol.

Fig. 1  Progesterone level after administration of 500 µg cloprostenol racemate

EP 0 295 619 A2

Fig. 2   Progesterone level after administration of 250 µg D-cloprostenol

EP 0 295 619 A2

Fig. 3  Progesterone level after administration of 250 µg L-cloprostenol

EP 0 295 619 A2

Fig. 4 Progesterone levels after administration of different doses of D-cloprostenol

EP 0 295 619 A2